# EUROPEAN PATENT APPLICATION

(11) **EP 2 267 459 A1**
(43) Date of publication of application: **29.12.2010**
(21) Application number: 09305601.8
(22) Date of filing: 25.06.2009
(51) Int. Cl.: G01N 33/68

(54) **Method for determining the susceptibility of a cell strain to drugs**

(71) Applicant: Universite Pierre Et Marie Curie - Paris VI, 75005 Paris (FR); Assistance Publique - Hôpitaux de Paris, 75001 Paris (FR)
(72) Inventor: Mazier, Dominique, 75010 Paris (FR); Marinach-Patrice, Carine, 91260 Juvisy sur Orge (FR); Alanio, Alexandre, 75015 Paris (FR); Golmard, Jean Louis, 75002 Paris (FR)
(74) Representative: Jacobson, Claude

(57) **Abstract**

The present invention relates to a method for determining the susceptibility of a cell strain to a compound intended for controlling the growth of said cell strain, comprising:
- growing the cell strain in a first compound-free culture medium and in at least a second culture medium comprising the compound at a test concentration;
- obtaining mass-spectrometry spectra for a protein extract of the cell strain grown in the first culture medium and for a protein extract of the cell strain grown in the second culture medium;
- comparing the mass spectrometry spectra;
- deducing that the cell strain is sensitive to the compound at the test concentration if the mass spectrometry spectra are significantly different.

## Description

### Field of the invention

The present invention relates to a method for determining the susceptibility of a cell strain to a compound intended for controlling the growth of said cell strain.

### Technical background

Testing the susceptibility of cell strains, such as tumour cell strains or microorganism strains, to drugs is a crude challenge, in particular in view of the increasing prevalence of drug resistance.

Indeed, besides cancer cells, resistances to drugs have notably been evidenced in bacteria, protozoan parasites, and fungi, such as yeasts and filamentous fungi. This has notably been evidenced in the case of the determination of the susceptibility of *Candida albicans* to fluconazole (FCZ). *Candida albicans* is the leading cause of invasive candidiasis, a major hospital-acquired infection. Fluconazole, an azole derivative agent, is one of the main first-line therapy alternatives. Azole resistant strains have emerged, possibly as a consequence of the use of azole-based antifungal agents in iterative and long-term therapies.

*In vitro* susceptibility testing is essential both for epidemiologic surveillance, *e.g*. to detect the emergence of resistant-microorganisms, and to adapt therapy for a given patient.

Susceptibility of cell strains to drugs is usually determined following the well-known broth microdilution methods as gold standards tests. These methods are based on growth inhibition and involve the determination of the Minimal Inhibitory Concentration (MIC). Such methods are notably recommended by the European Committee on Antibiotic Susceptibility Testing (EUCAST) and the Clinical Laboratory Standards Institute (CLSI) (Rodriguez-Tudela. et al. J Clin Microbiol 45, 109-111 (2007); Espinel-Ingroff et al. J Clin Microbiol 43, 3884-3889 (2005)).

For the EUCAST methodology, the MIC endpoint is determined as the drug concentration inducing a 50% growth inhibition (IC₅₀) with respect to the control as measured after 24h of growth with a spectrophotometer. For the CLSI methodology, MIC endpoints are defined visually as the point at which there is prominent reduction in growth in the sample as compared to the control, after 48 h of incubation. This visual end-point correlates with 50% growth inhibition (Rex et al. Clin Microbiol Rev 14, 643-658 (2001)).

Both reference methods are robust and reliable, though they remain time-consuming and thus inadequate for routine determination in an hospital context (Revankar et al. J Clin Microbiol 36, 153-156 (1998) ; Lass-Florl et al. Antimicrob Agents Chemother 52, 3637-3641 (2008)).

Thus, to circumvent this drawback, some commercial assays such as the E-Test (AB-Biodisk) or YeastOne Panel (Trek Diagnostic) have been proposed that can be used widely and easily in clinical microbiology labs. They have been favourably compared with the reference methods. However, they still remain quite long to carry out and reading of the assays may be particularly difficult. This is particularly the case when testing *C. albicans* isolates against fluconazole, since it frequently leads to a trailing phenomenon, defined by a paradoxical low-level growing of the isolates even over increasing concentrations of the drug. More recently, this so-called paradoxical effect has also been described for some *Candida* isolates when tested against ecchinocandin drugs.

Given these limitations, there is a clear need for the development of an equally robust method, with faster turn-around times and where endpoints determination is objective.

It is therefore an object of the present invention to provide such a method.

### Description of the invention

The present invention arises from the unexpected finding, by the inventors, that the protein composition of a *C. albicans* strain changes reproducibly in response to a particular drug concentration to which it is subjected, and that this variation in protein composition can be evidenced by mass spectrometry. Besides, the inventors have also shown that the values obtained for the minimal concentration of drug inducing a detectable change in mass spectrometry spectra of a protein extract of *C*. *albicans* with respect to that determined for *C. albicans.*

The present invention thus relates to a method for determining the susceptibility of a cell strain to a compound intended for controlling the growth of said cell strain, comprising:
- growing the cell strain in a first compound-free culture medium and in at least a second culture medium comprising the compound at a test concentration;
- obtaining mass-spectrometry spectra for a protein extract of the cell strain grown in the first culture medium and for a protein extract of the cell strain grown in the second culture medium;
- comparing the mass spectrometry spectra;
- deducing that the cell strain is sensitive to the compound at the test concentration if the mass spectrometry spectra are detectably different.

As intended herein, the expression "cell strain" relates to any kind of eukaryotic or prokaryotic cell strain. In particular, where the cell strain is an eukaryotic cell strain it can be from a pluricellular or an unicellular organism. As will be clear to one of skill in the art, the unicellular organism can notably such that it develops into a pluricellular organism. Preferably, the cell strain is a tumour cell strain or a microorganism strain. Preferably, the cell strain is a microorganism strain selected from the group constituted of a bacterial strain, a fungus strain, such as a filamentous fungus, in particular of the *Ascomycota* (*e.g.* of the *Aspergillus* or *Fusarium* genus) and *Zygomycota* phyla, or a yeast strain, in particular of the *Ascomycota* and *Basiodiomycota* phyla, a protozoan strain, and an algae strain. More preferably, the cell strain is a yeast strain, in particular selected from group consisting of a *Candida* strain, a *Saccharomyces* strain, a *Debaryomyces* strain, a *Pichia* strain, a *Geotrichum* strain, a *Cryptococcus* strain, a *Fisiobasidiella* strain, and a *Trichosporon* strain. Most preferably the cell strain is a *Candida* strain. Besides, among yeasts of the *Candida* genus, it is preferred that the cell strain is a *Candida* strain selected from a *Candida albicans* strain, a *Candida glabrata* strain, *a Candida tropicalis* strain, a *Candida parapsilosis* strain, a *Candida kefyr* strain, a *Candida krusei* strain, a *Candida dubliniensis* strain, a *Candida guillermondii* strain and a *Candida lusitaniae* strain, and particularly preferred that the cell strain is a *Candida albicans* strain.

As intended herein, the term "compound for controlling the growth of said cell strain" relates to a compound liable to kill cells of the cell strain or to inhibit, partially or totally, the growth of cells of the cell strain. Thus, the compound may notably be an anti-tumour compound, an antibiotic or antibacterial compound, or an antifungal compound. However, it is preferred that the compound is an antifungal compound selected from an azole compound, an echinochandin compound, such as caspofungin, micafungin, or anidulafungin, a polyene compound, such as amphotericin B or nystatin, and anti-metabolites, such as flucytosine. Preferably, the antifungal compound is an azole compound selected from the group constituted of fluconazole, voriconazole, posaconazole, isavuconazole, ravuconazole, ketoconazole, and itraconazole. Most preferably the compound is fluconazole.

As intended herein the expression "determining the susceptibility of a cell strain" relates to determining whether, and within what measure, the compound as defined above kills or inhibits the growth of cells of the cell strain. In particular, "determining the susceptibility of a cell strain" relates to determining the minimal concentration of the compound which yields a detectable difference in the mass spectrometry spectra.

As intended herein, "mass spectrometry" relates to any method enabling determining the m/z ratio of one or more molecules, such as proteins, within a sample, such as a protein extract as defined above, wherein m represents the mass and z the charge of said molecules. Mass spectrometry as defined above can be carried out by any one of the numerous mass spectrometry methods known in the art, such as Matrix-Assisted Laser Desorption/Ionisation Time-Of-Flight (MALDI-TOF) mass spectrometry, or Surface-Enhanced Laser Desorption/lonisation Time-Of-Flight (SELDI-TOF). However, it preferred that mass spectrometry as defined above is carried out by MALDI-TOF.

The expression "mass spectrometry spectra" relate to recordings of the m/z ratios and optionally the quantities of the various molecules, in particular proteins, contained in the protein extracts submitted to mass spectrometry. Usually, mass spectrometry spectra are graphs representing signal intensity (corresponding to the quantity of molecule) as a function of the m/z ratio. The association of signal intensity to an m/z ratio defines a peak. As will be clear to one of skill in the art "a mass spectrometry spectrum" as intended herein can be either obtained from one recording or be the mean of a plurality of recordings.

As intended herein, "mass spectrometry spectra are detectably different" in particular if a detectable difference in intensity of m/z ratio can be established. The man skilled in the art knows how to establish that two spectra present detectable differences, in particular using exact permutation tests based on Spearman rank correlation coefficients, such as described in "Design and Analysis of DNA Microarray Investigations", RM Simon et al, SPRINGER, 2003, in particular on pages 68 and 123.

Numerous procedures are known in the art for extracting proteins from cells and one of skill in the art knows how to adapt them depending on the type of cell strain. Accordingly, any one of these extraction methods can be used in the method of the invention. However, it is preferred, within the frame of the method according to the invention, that the protein extract is obtained by an ethanol treatment of grown cells followed by a treatment with a mixture of formic acid and acetonitrile, in particular where the cell strain is a yeast strain, more particularly a *Candida* strain.

As intended herein, the expression "culture medium" relates to any medium liable to sustain the growth of cells of the cell strain. Preferably, the culture medium as defined above is a minimum medium. Preferably also, the medium is a liquid medium. As will be clear to one of skill in the art, the composition of the compound-free culture medium and the culture medium comprising the compound at a test concentration should preferably identical except for said compound.

The cell strain can be grown for any amount of time provided it is sufficient for the compound to induce significant changes in the protein content of the cell strain. However, so that the method is carried out as quickly as possible, it is preferred that the amount of time for growing the cells is the minimal time for the compound to induce significant changes in the protein content of the cell strain. Thus, the cell strain is grown during less than 24 hours, more preferably during less than 20 hours, and most preferably during about 15 hours.

In an embodiment of the above-defined method, the cell strain is grown in several culture media with increasing test concentrations of the compound, and the minimal concentration of the compound yielding a mass-spectrometry spectrum detectably different from the mass-spectrometry spectrum obtained from the cell-extract of the microorganism strain grown in the compound-free culture medium is determined.

As intended herein the "minimal concentration of the compound yielding a mass-spectrometry spectrum significantly different from the mass-spectrometry spectrum obtained from the cell-extract of the microorganism strain grown in the compound-free culture medium" is also called the minimal profile (*i.e.* mass spectrometry spectrum) change concentration (MPCC). Advantageously, the inventors have shown that for a given cell strain and compound the MPCC and the MIC are correlated.

Preferably, determining the minimal concentration comprises:
- obtaining a mass spectrometry spectrum from a protein extract of the cell strain grown in the culture medium having the highest test concentration of the several culture media;
- comparing the mass spectrometry spectra obtained from protein extracts of the several culture media to (i) the mass spectrometry spectrum from a protein extract of the cell strain grown in the culture medium having the highest test concentration and (ii) the mass spectrometry spectrum from the compound-free culture medium;
- selecting the mass spectrometry spectrum obtained from a protein extract of the several culture media with the lowest compound concentration and which presents more similarity with the mass spectrometry spectrum (i) than with the mass spectrometry spectrum (ii);
the lowest compound concentration being the minimal concentration to be determined.

Determining that a mass spectrometry spectrum presents more similarity, or shares more resemblance, with a first spectrum than with a second spectrum can be routinely determined by one of skill in the art, in particular using a similarity measure based on Spearman rank correlation coefficients, and more particularly as described in the following Examples.

The invention will be further described by the following non-limiting figures and Examples.

### Description of the figures

Figure 1 represents alterations in the mass spectra of the DSY2260 *C. albicans* strain exposed to increasing fluconazole (FCZ) concentrations (virtual gel). The x-axis represents m/z value, on the left the y-axis shows running spectrum number, while on the right peak intensity is expressed in a grey colour scale with arbitrary units (a.u.). Briefly, 10⁶ yeasts/ml were cultured for 15h, with three biological replicates for each FCZ concentration (from 128 to 0.125 µg/ml) and for unexposed yeasts. Acidic extracts were analysed in duplicate by MALDI-TOF MS.
Figures 2 and 3 represent the average mass spectra of the 6 replicate spectra of DSY2260 *C. albicans* exposed to 2 µg/ml of FCZ (figure 2) and 4 µg/ml of FCZ (figure 3) (mass range 5800-7600 m/z).
Figure 4 represents the correlation between MICs evaluated by the CLSI methodology and MPCCs determined by a MALDI-TOF MS method according to the invention (regression line).

### EXAMPLE

### 1. Methods

### Yeasts strains

All experiments were performed using sequential *Candida albicans* clinical isolates. Eight groups of sequential related isolates (groups A to K) were characterized. All strains were isolated from HIV-positive patients with oropharyngeal candidasis that were treated mainly with FCZ. Each group contains one azole susceptible strain (FCZ MIC ranged from 0.125 to 8 µg/ml) and its related-sequential strains with higher FCZ MICs (ranging from 16 to 128 µg/ml) within the same MLST genotype. For each clinical isolate, MICs according to CLSI broth-microdilution methodology1, and presence of *ERG11* and *TAC* mutations, and/or *CDR1*/*2* and *MDR* hyperexpression were determined previously2 **(Table 1)**. The ATCC 90028 *C*. *albicans* strain (MIC=0.25 µg/ml) was added as a reference strain.

### Antifungal agents

Fluconazole (FCZ) pure powder (Sigma Chemical CO., Saint-Louis, MO, USA) was dissolved in pure water. Serial dilutions of drug (concentration ranged from 256 to 0.25 µg/ml), made into RPMI 1640 medium (with glutamine and without bicarbonate, Invitrogen) buffered with MOPS (0.165M) (Sigma Chemical CO., Saint-Louis, MO, USA) and adjusted to pH 7 with sodium hydroxide (5N), were dispensed in 600 µl aliquots into sterile 24-well flat-bottomed microtiter plates.

### Samples preparation

After cultivation during 48h at 37°C on Sabouraud agar medium, yeasts cells were transferred into RPMI 1640 medium (with glutamine but without bicarbonate, Invitrogen) buffered with MOPS (0.165M) (Sigma Chemical CO., Saint-Louis, MO, USA), and adjusted to pH 7 with sodium hydroxide (5N). Then, 600 µl of RPMI with yeast (2.106 yeasts/ml) were added to 600 µl of RPMI with FCZ into microtiter plates (final FCZ concentration ranged from 128 to 0.125 µg/ml), or in RPMI alone as negative control. Culture was performed in a 30°C incubator for 15 hours, with continuous agitation. Yeast extraction was performed as follows:
(1) samples were centrifuged and the supernatant removed before the pellet was washed twice in pure water;
(2) the pellet was then resuspended in 300 µL of pure water, and 900 µL of ethanol were then added;
(3) after another round of centrifugation, 50 µL of 70% formic acid and 50 µL pure acetonitrile were added to the residual pellet and subsequent solution was repeatedly and thoroughly vortexed before a final centrifugation.
Each centrifugation was performed at 10 000rpm for 10 minutes at room temperature.

### MALDI-TOF mass spectrometry

The supernatant was distributed (0.5 µl droplet) in duplicate on a MALDI AnchorChip sample slide (Bruker-Daltonics, Bremen, Germany), then air-dried. The α-cyano-4-hydroxycinnamic acid (CHCA) matrix (Bruker-Daltonics), prepared at a concentration of 50 mg/ml in 50% acetonitrile and 50% water with 0.1% TFA, was sonicated for 5 min before being spotted (0.5 µl) over the dried sample. A DH5a *Escherichia coli* protein extract (Bruker-Daltonics) was deposited on the calibration spot of the Anchorchip for external calibration. MALDI analysis were performed on a Bruker Autoflex I MALDI TOF mass spectrometer with a nitrogen laser (337 nm) operating in linear mode with delayed extraction (260 ns) at 20 kV accelerating voltage. Each spectrum was automatically collected in the positive ion mode as an average of 500 laser shots (50 laser shots at 10 different spot positions). Laser energy was set just above the threshold for ion production. Mass range between 3,000 and 20,000 m/z (ratio mass/charge) was selected to collect the signals with the AutoXecute tool of flexControl acquisition software (Version 2.4; Bruker-Daltonics). Only peaks with a signal/noise ratio >3 were considered. Spectra were eligible for further analysis when the peaks had a resolution better than 600. For each cultivation condition, we collected mass spectra from 3 biological replicates and 2 technical replicates.

### Statistical analysis

### a) Pre-processing ending

Data obtained from the flex control acquisition software were a set of lists of mass/charge peaks and corresponding intensities, one list for each spectrum. For each FCZ concentration (11 FCZ concentrations and without FCZ), 6 spectra, obtained from 3 biological replicates tested in duplicate, were collected, so the whole initial set of data consisted of 72 spectra.

Alignment was performed using a method based on the mean spectrum, using an approach similar to that recommended by Coombes et al. (Coombes KR, Baggerly KA, and Morris JS: Pre-Processing Mass Spectrometry Data. Fundamentals of Data Mining in Genomics and Proteomics, W Dubitzky, M Granzow, and D Berrar, eds. Kluwer, Boston, 79-99 (2007)).

The mean spectrum of the 72 spots was computed, a simple peak detection algorithm was run on the mean spectrum, and a selection of the final peak locations was based on the mean intensity of the peak. For each spectrum, the peaks corresponding to the final peak locations were retained for the statistical analysis. An error of 0.05 % of the mass/charge ratio was allowed in the two last steps.

### b) Comparison between extreme concentrations

At this stage, data consisted of 12 ordered subsets of 6 spectra, each subset corresponding to a concentration value, from 0 to 128 µg/ml. The first step of statistical analysis was aimed at testing whether there was a difference between the extreme concentration spectra.

An exact permutation test using Spearman rank correlation coefficient as a similar measure, based on the following computations, has been carried out:
- in a first step, all the rank correlation coefficients between the 12 spectra were computed;
- in a second step, the mean of the intra-class rank correlations coefficients (IntraRCCM) and the mean of the inter-class rank correlation coefficients (InterRCCM) were computed.

The test criterion is the ratio InterRCCM/IntraRCCM Under the null hypothesis of no difference between class memberships, the criterion's expected value is 1. When class memberships are informative, interRCCM is lower than intraRCCM, and expected criterion values are lower than 1. The permutation test is achieved by computing the distribution of the criterion for all the permutations of the class memberships, and the exact p-value is the proportion of criteria lower or equal to the one corresponding to the observed criterion value.

### c) Determination of the MPCC

Once the difference between extreme concentrations has been statistically proved, the aim of the next step is to find the minimal concentration at which a particular spectrum starts to differ significantly from the null control spectrum one. This is achieved by computing for each concentration, the corresponding spectrum similarity with each from the two extreme concentrations, and by classifying it as "near of the null concentration" or "near of the maximum concentration" according to the similarity values. The similarity function used is the mean inter-class rank correlation coefficient (InterRCCM). The minimal profile change concentration (MPCC) is defined as the minimum concentration that is more similar to the maximum concentration than to the null one.

### 2. Results

In order to obtain standardized fingerprints, the inventors first optimized the protocols. All experiments were carried out using the *C. albicans* susceptible reference strain, ATCC 90028 **(Methods).** The influence of starting inoculum, the concentration of yeast cells in the sample to be analyzed and thus the minimum culturing time required, on the quality and reproducibility of the fingerprint reproducibility were assessed. This allowed establishing that optimal results are obtained from cultures initiated with 10⁶ yeasts/ml.

The inventors then determined the effect that varying concentrations of FCZ (serial dilution from 128 to 0.125 µg/ml) would have on *C. albicans* mass spectrometry fingerprint patterns **(Methods).**

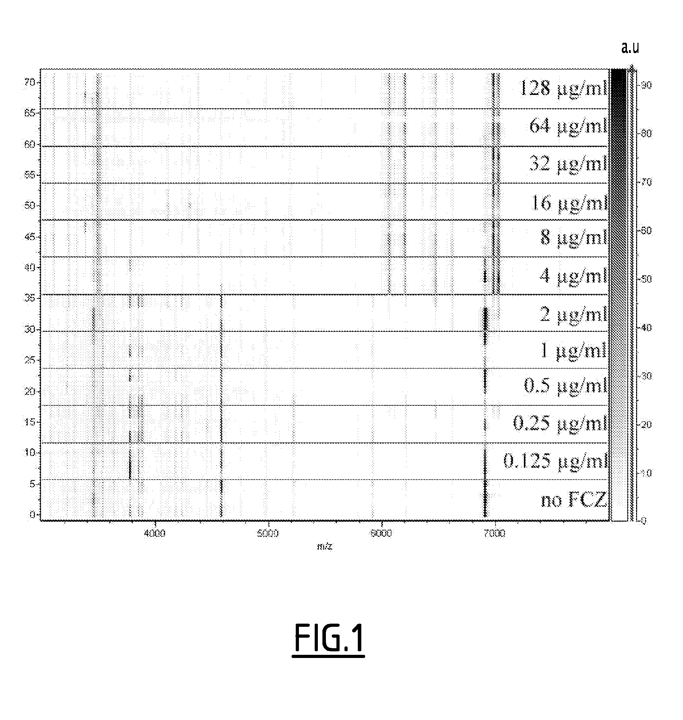

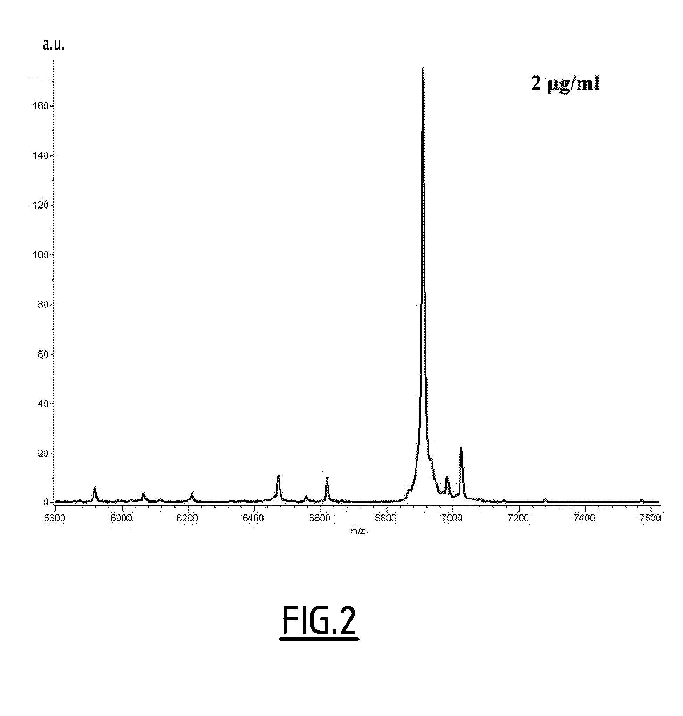

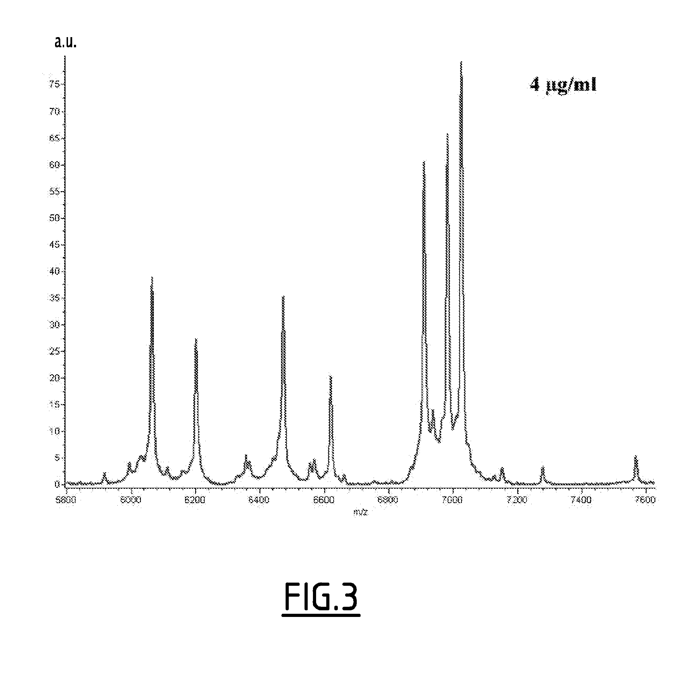

The yeast cell pellets obtained from cultures grown for 15 h were subjected to acid extraction and the supernatants analyzed by MALDI-TOF MS. A typical result is presented in **Fig. 1** where the mass spectrometry spectra of the DSY2260 clinical *C*. *albicans* strain (FCZ MIC = 8 µg/ml) from cultures exposed to serial FCZ dilutions are shown. For this strain, the mass spectrometry spectra from cultures exposed up to 2 µg/ml FCZ are indistinguishable from those observed for the control culture. Conversely, the spectra deviate from the control in a detectable and quantifiable manner (through gain or loss of spectra) in the cultures exposed to 4 µg/ml and upwards **(****Fig. 2** **and** **3****).**

Accordingly, these observations led the inventors to formulate a new endpoint, namely the minimal profile change concentration (MPCC), a value defined as the lowest drug (FCZ in the experiment above) concentration at which a mass spectrum profile change can be detected.

Besides, the inventors have further devised a novel statistical approach to calculate this value objectively **(Methods),** which is based on the mass and intensity of each peak in the fingerprints. In this statistical analysis, the discrepancies between the mass spectra at the two extreme conditions (128 µg/ml FCZ and no FCZ) are first defined. Then, the similarity to each of the two "extreme" spectra is statistically evaluated for the spectrum recorded at each of the different intermediate FCZ concentrations, to yield a classification of "nearer to the 128 µg/ml" or "nearer to the FCZ negative" spectrum.

The validity of the new methodology above was established as follows. The MPCC of the reference strain (*C. albicans* ATCC 90028, MIC = 0.25 µg/ml) was determined, as well as that of sixteen *C. albicans* isolates with distinct drug resistance profiles. Eight are known to have low-MICs (range 0.125 to 8 µg/ml) and the other eight have high-MICs (range 16 to 128 µg/ml) to FCZ, these MICs having been determined by the CLSI standard method. In addition, the FCZ-resistant strains tested are representative of the different resistance mechanisms, mating types and clades15 that are found in *C. albicans* **(Table 1)**.

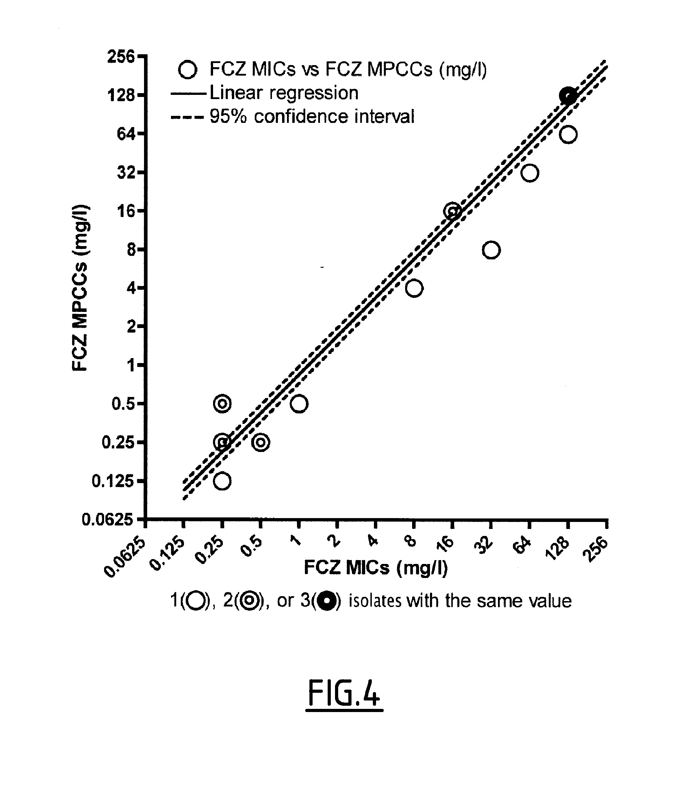

Comparison of MPCCs with the MICs for the 17 strains above **(****Fig. 4** and **Table 1)** revealed a very high degree of concordance, with full agreement for 94% and 100% of the strains, depending on whether concordance was scored as +/-1 or within +/- 2 drug dilution values, respectively. A cut-off set at +/- 2 drug dilution values is the maximum accepted discrepancy for agreement in comparison conducted between antifungal susceptibility testing methods (Espinel-Ingroff et al. J. Clin Microbiol 43, 3884-3889 (2005)).

Importantly, the MPCC determinations were concordant and accurate irrespective of the type of drug resistance mechanism (*ERG11* mutations, *TAC* mutation, *CDR1*/*2* or *MDR* hyper-expression), the mating type, or the clade to which the different strains tested belonged. These results not only validate the new methodology, but also offer strong indication of the robustness of the methodology present in the face of *C. albicans* strains with diverse genetic backgrounds.

As it stands, where a mass spectrometer is already available, the running costs for each sample analyzed by the method presented here is slightly less than 1 Euro, which compares quite favourably with the higher costs of all other methods. The diagnostic profile shift observed for FCZ does not vary with either the genetic background of the strain tested, nor the level or mechanism of the resistance to FCZ. Accordingly, it is likely that a characteristic profile would be associated with each of the different classes of drugs known to inhibit a given pathogen (*e.g*. triazoles echinocandins, polyene, and antimetabolites for *C. albicans*). The present methodology appears to be suitable for monitoring the emergence of resistance to drugs used against pathogenic organisms, including bacteria and eukaryotic cancer cells or pathogens such as *Cryptosporidium* and *Plasmodium.*

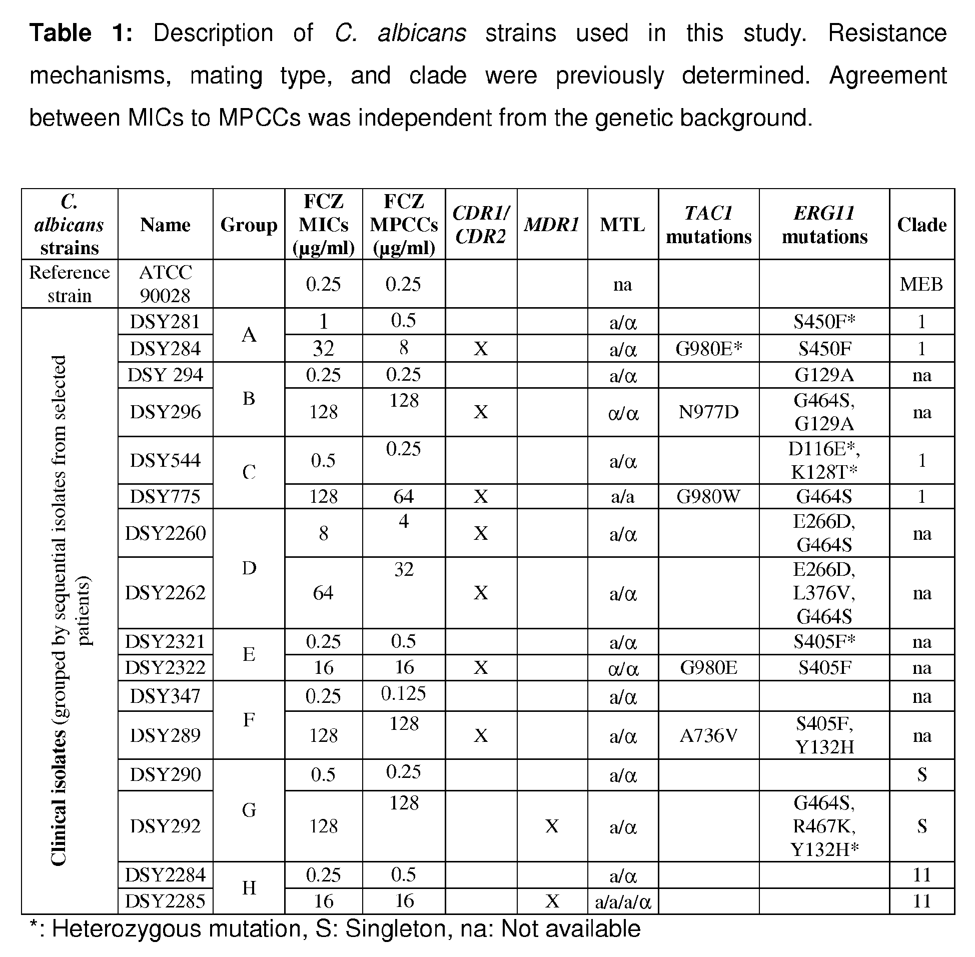

## Claims

1. A method for determining the susceptibility of a cell strain to a compound intended for controlling the growth of said cell strain, comprising:
- growing the cell strain in a first compound-free culture medium and in at least a second culture medium comprising the compound at a test concentration;
- obtaining mass-spectrometry spectra for a protein extract of the cell strain grown in the first culture medium and for a protein extract of the cell strain grown in the second culture medium;
- comparing the mass spectrometry spectra;
- deducing that the cell strain is susceptible to the compound at the test concentration if the mass spectrometry spectra are significantly different.

2. The method according to claim 1, wherein the cell strain is a tumour cell strain or a microorganism strain.

3. The method according to claim 1 or 2, wherein the cell strain is a microorganism strain selected from the group constituted of a bacterial strain, a fungus strain, a protozoan strain, and an algae strain.

4. The method according to any of claims 1 to 3, wherein the cell strain is a yeast strain.

5. The method according to any of claims 1 to 4, wherein the cell strain is a *Candida* strain.

6. The method according to any of claims 1 to 5, wherein the cell strain is a *Candida* strain selected from a *Candida albicans* strain, a *Candida glabrata* strain, *a Candida tropicalis* strain, a *Candida parapsilosis* strain, a *Candida kefyr* strain, a *Candida krusei* strain, a *Candida dubliniensis* strain, a *Candida guillermondii* strain and a *Candida lusitaniae* strain.

7. The method according to claim 1 to 6, wherein the cell strain is a *Candida albicans* strain.

8. The method according to any of claims 1 to 7, wherein the compound is an antifungal compound.

9. The method according to any of claims 1 to 8, wherein the compound is an antifungal azole compound selected from the group constituted of fluconazole, ketoconazole, itraconazole, voriconazole, posaconazole, isavuconazole, and ravuconazole.

10. The method according to any of claims 1 to 9, wherein the compound is fluconazole.

11. The method according to any of claims 1 to 10, wherein mass spectrometry is carried out by MALDI-TOF.

12. The method according to any of claims 1 to 11, wherein the protein extract is obtained by an ethanol treatment of grown cells followed by a treatment with a mixture of formic acid and acetonitrile.

13. The method according to any of claims 1 to 12, wherein the culture medium is a minimum medium.

14. The method according to any of claims 1 to 13, wherein the cell strain is grown during 16 hours.

15. The method according to any of claims 1 to 14, wherein the cell strain is grown in several culture media with increasing test concentrations of the compound, and the minimal concentration of the compound yielding a mass-spectrometry spectrum detectably different from the mass-spectrometry spectrum obtained from the cell-extract of the microorganism strain grown in the compound free culture medium is determined.

16. The method according to claim 15, wherein determining the minimal concentration comprises:
- obtaining a mass spectrometry spectrum from a protein extract of the cell strain grown in the culture medium having the highest test concentration of the several culture media;
- comparing the mass spectrometry spectra obtained from protein extracts of the several culture media to (i) the mass spectrometry spectrum from a protein extract of the cell strain grown in the culture medium having the highest test concentration and (ii) the mass spectrometry spectrum from the compound-free culture medium;
- selecting the mass spectrometry spectrum obtained from a protein extract of the several culture media with the lowest compound concentration and which presents more similarity with the mass spectrometry spectrum (i) than with the mass spectrometry spectrum (ii);
the lowest compound concentration being the minimal concentration to be determined.
